# EUROPÄISCHE PATENTSCHRIFT

(11) **EP 0 981 995 B1**
(45) Veröffentlichungstag und Bekanntmachung des Hinweises auf die Patenterteilung: **31.08.2005**
(21) Anmeldenummer: 99202483.6
(22) Anmeldetag: 28.07.1999
(51) Int. Cl.: A61B 6/03, G01N 23/04

(54) **Computertomograph mit kegelförmigem Strahlenbündel und helixförmiger Abtastbahn**
Computer tomograph with conical beam and helical source trajectory
Tomographe assisté par ordinateur avec un faisceau de rayonnement conique et un balayage hélicoidal

(30) Priorität: 05.08.1998 DE 19835296
(43) Veröffentlichungstag der Anmeldung: 01.03.2000
(73) Patentinhaber: Philips Intellectual Property & Standards GmbH, 20099 Hamburg (DE); Koninklijke Philips Electronics N.V., 5621 BA Eindhoven (NL)
(72) Erfinder: Proksa, Roland, Röntgenstrasse 24, 22335 Hamburg (DE); Timmer, Jan, Röntgenstrasse 24, 22335 Hamburg (DE)
(74) Vertreter: Volmer, Georg

(56) Entgegenhaltungen:
- WO-A-98/30980
- US-A- 5 291 402
- KUDO H ET AL: "Cone-beam filtered-backprojection algorithm for truncated helical data" PHYSICS IN MEDICINE AND BIOLOGY, TAYLOR AND FRANCIS LTD. LONDON, GB, Bd. 43, Nr. 10, Oktober 1998 (1998-10), Seiten 2885-2909, XP002102880 ISSN: 0031-9155

## Beschreibung

Die Erfindung betrifft einen Computertomographen mit
- einer Abtasteinheit, die eine Strahlenquelle und eine damit verbundene Detektoreinheit zur Erfassung eines von der Strahlenquelle emittierten, kegelförmigen Strahlenbündels nach dem Durchgang durch einen Untersuchungsbereich bzw. durch ein darin befindliches Objekt umfaßt,
- einer Antriebsanordnung zur Erzeugung einer eine Rotation um eine Rotationsachse (14) und einen Vorschub in Richtung parallel zur Rotationsachse umfassenden Relativbewegung zwischen der Abtasteinheit und dem Untersuchungsbereich bzw. dem Objekt in Form einer Helix (17)
- und mit einer Rekonstruktionseinheit zur Rekonstruktion der räumlichen Verleitung der Absorption innerhalb des Untersuchungsbereiches aus den von der Detektoreinheit innerhalb eines durch die Helix definierten Detektorfensters akquirierten Meßdaten.

Ein solcher Computertomograph (im folgenden auch CT-Gerät genannt) ist aus der WO 98/30980 bekannt. Bei dem bekannten Computertomographen werden zur Rekonstruktion der Absorptionsverteilung nur diejenigen Meßdaten herangezogen, die sich innerhalb eines Detektorfensters befinden, das in Richtung der z- Achse durch die Projektion zweier aufeinanderfolgender Windungen der Helix definiert ist (als Detektorfenster wird hierbei und im folgenden der Teil der Meßfläche der Detektoreinheit bezeichnet, der die für die Rekonstruktion erforderlichen Daten - und nur diese- erfaßt). Es läßt sich zeigen, daß bei einer solchen Gestaltung des Detektorfensters die Strahlenquelle jedes Voxel im Untersuchungsbereich bei seinem Eintritt und bei seinem Austritt aus dem Strahlenbündel aus um exakt 180° versetzten Positionen (bezogen auf das jeweiligen Voxel) auf das Detektorfenster projiziert wird. Die auf diese Weise erhaltenen Meßdaten erlauben eine exakte Rekonstruktion der Absorptionsverteilung im Untersuchungsbereich und zwar auch dann, wenn das darin enthaltene Objekt länger ist als der vom kegelförmigen Strahlenbündel erfaßte Teil des Untersuchungsbereichs.

Aufgabe der vorliegenden Erfindung ist es, einen Computertomographen der eingangs genannten Art noch weiter zu verbessern. Diese Aufgabe wird erfindungsgemäß dadurch gelöst, daß die Verbindungsgeraden der Strahlenquelle mit den beiden in Richtung der Rotationsachse gegeneinander versetzten Rändern des Detektorfensters zwei in Richtung der Rotationsachse um die Strecke (2n+1)p versetzte Abschnitte der Helix schneiden, wobei n eine ganze Zahl ≥ 1 ist und p dem achsialen Versatz zweier benachbarter Windungen der Helix entspricht.

Während bei dem bekannten Computertomographen die beiden Ränder des Detektorfensters voneinander einen Abstand haben, der der Projektion zweier benachbarter Detektorwindungen auf die Detektoreinheit entspricht, beträgt bei der Erfindung dieser Abstand ein ungeradzahliges Vielfaches davon. Das hat zur Folge, daß dann, wenn die Projektion eines Punktes im Untersuchungsbereichs von einem Rand des Fensters zum anderen Rand gewandert ist, die Strahlenquelle sich um diesen Punkt genau um einen Winkel (2n+1)π gedreht hat. Auch in diesem Fall ergeben sich keine redundanten Meßdaten. Alle innerhalb des Detektorfensters akquirierten Meßdaten können zur Rekonstruktion herangezogen werden.

Die Tatsache, daß sich die Strahlenquelle um die einzelnen Punkte des Untersuchungsbereichs um ein ungeradzahliges Vielfaches dreht, hat zur Folge, daß die Empfindlichkeit des Verfahrens gegenüber Dateninkonsistenz (die aus Bewegung des Untersuchungsobjekts während der Abtastung resultieren) geringer ist als bei dem bekannten Verfahren. Dies obwohl das Singnal/Rauschverhältnis - über den gesamten Untersuchungsbereich gemittelt - prinzipiell nicht schlechter ist, als bei der Akquisition der Meßdaten mit dem bekannten Computertomographen (vorausgesetzt, daß die Vorschubgeschwindigkeit und die Intensität der Strahlenquelle in beiden Fällen gleich sind).

Das Signal/Rauschverhältnis in einzelnen Punkten ist jedoch gleichmäßiger über den Untersuchungsbereich verteilt als bei dem bekannten Computertomographen, weil die Zeiträume, während denen ein Punkt auf das Detektorfenster projiziert werden, weniger stark ortsabhängig schwanken. Bei dem bekannten Computertomographen ergibt sich eine Variation der Projektionsdauer von 2:1, wenn der Radius des Objektzylinders, innerhalb dessen ein Objekt vollständig von dem kegelförmigen Strahlenbündel erfaßt werden kann, halb so groß ist wie der Radius der Bahn der Strahlenquelle um die Rotationsachse. Bei einem erfindungsgemäßen Gerät beträgt diese Variation bei gleicher Geometrie nur 1,25:1 (für n=1) bzw. 1,14:1 (für n=2). Die mit dieser Variation einhergehenden Artefakte werden durch die Erfindung also deutlich reduziert.

Das erfindungsgemäße Derektörfehster kann dadurch realisiert werden, daß die Detektoreinheit und/oder das von der Strahlenquelle durch einen Kollimator ausgeblendete kegelförmige Strahlenbündel entsprechend geformt werden. Wenn die Detektoreinheit einen Kreisbogen um die Rotationsachse, müßte die Abwicklung des Detektorfensters die Form eines Parallelogramms haben; ein verzerrtes Parallelogramm wäre nötig, wenn die Detektoreinheit (in einer zur Rotationsachse senkrechten Ebene) einen Kreisbogen um die Strahlenquelle definiert. Detektoreinheiten mit solchen Formen der Abwicklung sind aufwendig zu realisieren.

Man kann jedoch auch eine Detektoreinheit verwenden, deren Abwicklung die Form eines Rechtecks hat, wenn man dieses Rechteck in Richtung der Rotationsachse so groß macht, daß sie die Abwicklung des Detektorfensters umschließt. Ein solcher rechteckiger Detektor muß also größer sein als das gewünschte Detektorfenster. Bei der Erfindung ist das Verhältnis zwischen der benötigten Detektorfläche und der tatsächlichen Detektorfläche aber günstiger als bei dem Detektorfenster des bekannten CT-Gerätes.

Eine bevorzugte Weiterbildung ist in Anspruch 2 angegeben. In diesem Fall beträgt der Abstand zwischen den Rändern des Detektorfensters also das 3- fache des Abstandes zweier Windungen der Helix.

Eine bevorzugte Weiterbildung ist in Anspruch 3 angegeben. Die dadurch erreichten Vorteile sind analog denen, die bei konventionellen Computertomographen mit nur einer einzigen Detektorzeile auftreten, bei der die Mitte der Detektorelemente und der Durchstoßpunkt einer die Rotationsachse schneidenden Geraden um eine Viertel Detektorbreite gegeneinander versetzt sind: Nach einer halben Umdrehung der Abtasteinheit ist die Detektoreinheit in Zeilenrichtung um eine halbe Detektorbreite verschoben. Deshalb werden dieselben Daten nicht zweimal gemessen, sondern in Zwischenpositionen, weshalb die Meßdaten günstiger verteilt sind und sich die Bildqualität verbessert. Bei dem bekannten Computertomographen ist ein solcher Versatz nicht möglich.

Ähnliche Effekte ergeben sich mit der Weiterbildung nach Anspruch 4 auch in Richtung der Rotationsachse.

Die in Anspruch 5 beschriebene Ausgestaltung der Erfindung gestattet es, zwischen einem (ersten) Betriebsmodus zu wählen, bei dem sich die Strahlenquelle um jeden Untersuchungspunkt während seines Durchgangs durch den Strahlenkegel um den Winkel π dreht und einem (zweiten) Beiriebsmodus in dem diese Drehung (2n+1)π ist. Legt man für beide Betriebsmoden die gleiche Rotationsgeschwindigkeit zugrunde, dann ist - bei gleichen Detektorabmessungen - die Abtastgeschwindigkeit im ersten Betriebsmodus um den Faktor 2n+1 größer als im zweiten Betriebsmodus, in dem dafür das Signal/Rauschverhältnis bzw. die Bildqualität besser ist. Der Benutzer kann hierbei also den für die jeweilige Untersuchung günstigeren Betriebsmodus wählen.

Eine bevorzugte Ausgestaltung zur Rekonstruktion der Absorptionsverteilung in dem Untersuchungsbereich aus den innerhalb des Detektorfensters akquirierten Meßdaten ist in Anspruch 6 beschrieben. Man kann aus den Meßdaten die Absorptionsverteilung zwar auch auf andere Weise rekonstruieren, jedoch ergeben sich mit den angegebenen Mitteln besonders einfache Rekonstruktionsschritte und eine besonders gute Qualität der Rekonstruktion bzw. eine besonders gute Bildqualität.

Die Erfindung wird nachstehend anhand von Zeichnungen näher erläutert. Es zeigen:
Fig. 1 einen erfindungsgemäßen Computertomographen in schematischer Darstellung,
Fig. 2 die helixförmige Abtastbahn, die die Abtasteinheit und ein im Untersuchungsbereich befindliches Objekt relativ zueinander beschreiben,
Fig.3 eine Abwicklung der Detektoreinheit,
Fig. 4 die geometrischen Verhältnisse, in Richtung der Rotationsachse gesehen,
Fig. 5 eine andere Detektoreinheit in einem ersten Betriebsmodus,
Fig. 6 die gleiche Detektoreinheit in einem zweiten Betriebsmodus,
Fig. 7 die Anordnung einzelner Detektorelemente innerhalb der Detektoreinheit,
Fig. 8 ein Abiaufdiagramm zur Verarbeitung der Meßdaten, '
Fig. 9 die gleiche Ansicht wie Fig. 4, jedoch nach einem teilweisen Rebinning.

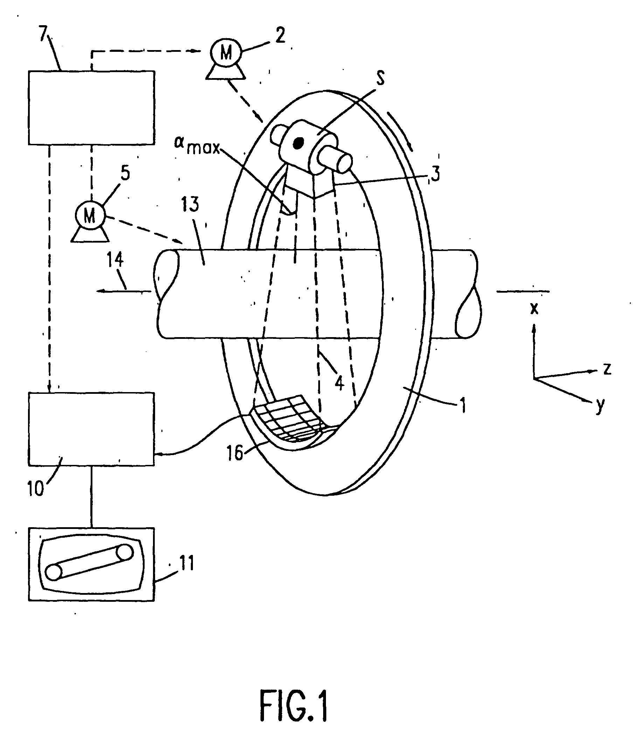

Der in Fig. 1 dargestellte Computertomograph umfaßt eine Gantry 1, die um eine parallel zur z-Richtung verlaufende Rotationsachse 14 rotieren kann. Dazu wird die Gantry von einem Motor 2 mit einer vorzugsweise konstanten, aber einstellbaren Winkelgeschwindigkeit angetrieben. An der Gantry ist eine Strahlenquelle S, beispielsweise eine Röntgenröhre, befestigt. Diese ist mit einer Kollimatoranordnung 3 versehen, die aus der von der Strahlenquelle S erzeugten Strahlung ein kegelförmiges Strahlenbündel 4 ausblendet, d.h. ein Strahlenbündel, das sowohl in Richtung der z-Achse als auch in einer dazu senkrechten Richtung (d.h. in der x-y-Ebene des in Fig. 1 dargestellten Koordinatensystems) eine von Null verschiedene, endliche Ausdehnung hat.

Das Strahlenbündel 4 durchdringt ein nicht näher dargestelltes Objekt, das sich in einem Untersuchungsbereich 13 befindet. Der Untersuchungsbereich 13 hat die Form eines Zylinders, der im folgenden auch als Objektzylinder bezeichnet wird. Nach dem Durchsetzen des Objektzylinders 13 trifft das Röntgenstrahlenbündel 4 auf eine an der Gantry 1 befestigte zweidimensionale Detektoreinheit 16, die eine Anzahl von Detektorzeilen mit jeweils einer Vielzahl von Detektorelementen umfaßt. Jedes Detektorelement erfaßt in jeder Strahlenquellenposition einen Strahl aus dem Strahlenbündel 4. Die Detektoreinheit 16 kann auf einem Kreisbogen um die Rotationsachse 14 angeordnet sein, jedoch sind auch andere Detektorgeometrien möglich, z.B. die Anordnung auf einem Kreisbogen um die Strahlenquelle S.

Der mit αₘₐₓ bezeichnete Öffnungswinkel des Strahlenbündels 4 (als Öffnungswinkel ist der Winkel definiert, den ein in der x-y-Ebene am Rande liegender Strahl des Bündels 4 mit einer durch die Strahlenquelle S und die Rotationsachse 14 definierten Ebene einschließt) bestimmt dabei den Durchmesser des Objektzylinders 13, innerhalb dessen das zu untersuchende Objekt sich bei der Akquisition der Meßwerte befinden muß. Der Untersuchungsbereich 13 - bzw. ein darin befindliches Objekt, beispielsweise ein auf einem Patientenlagerungstisch befindlicher Patient - kann mittels eines Motors 5 parallel zur Richtung der Rotationsachse 14 bzw. der z-Achse verschoben werden. Die , Geschwindigkeit dieses Vorschubs in z-Richtung ist konstant und vorzugsweise einstellbar.

Die von der Detektoreinheit 16 akquirierten Meßdaten werden einem Bildverarbeitungsrechner 10 zugeführt, der daraus die Absorptionsverteilung in dem vom Strahlenkegel 4 erfaßten Teil des Untersuchungsbereichs 13 rekonstruiert und z.B. auf einem Monitor 11 wiedergibt. Die beiden Motoren 2 und 5, der Bildverarbeitungsrechner 10, die Strahlenquelle S und der Transfer der Meßdaten von der Detektoreinheit 16 zum Bildverarbeitungsrechner 10 werden von einer geeigneten Kontrolleinheit 7 gesteuert.

Wenn der Motor 5 stillsteht und der Motor 2 die Gantry rotieren läßt, ergibt sich eine kreisförmige Abtastbewegung der Strahlenquelle S und der Detektoreinheit. Die Kontrolleinheit 7 kann die Motoren 2 und 5 aber auch so steuern, daß das Verhältnis der Vorschubgeschwindigkeit v des Untersuchungsbereichs 13 und die Winkelgeschwindigkeit ω der Gantry in einem konstanten Verhältnis stehen. In diesem Fall bewegen sich die Strahlenquelle S und der Untersuchungsbereich relativ zueinander auf einer helixförmigen Bahn. Im folgenden soll nur diese helixförmige Abtastbewegung betrachtet werden. Im Prinzip ist es bei einer helixförmige Abtastbewegung gleichgültig, ob die Abtasteinheit S, 16 bzw. der Untersuchungsbereich 13 die Rotations- bzw. Vorschubbewegung ausführen; wesentlich ist allein die Relativbewegung.

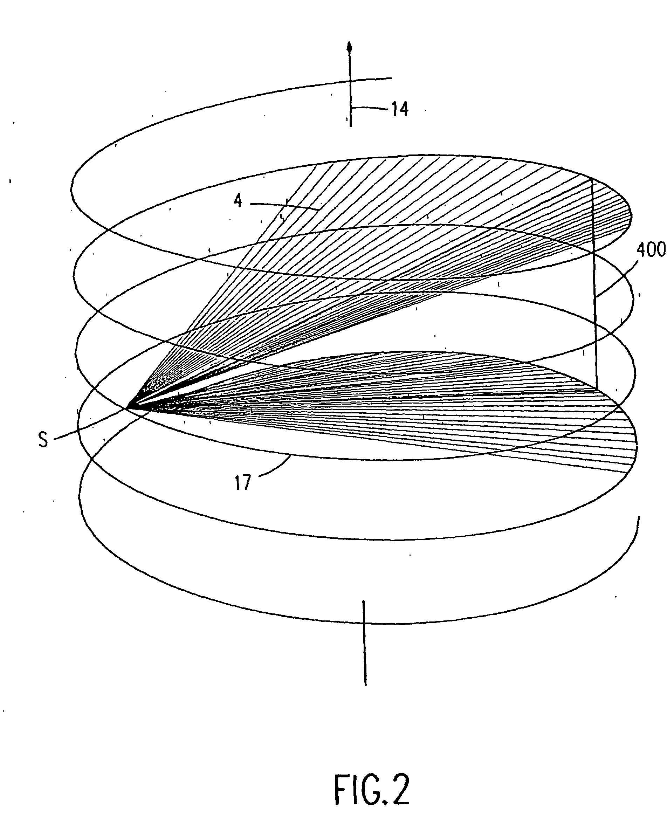

Deshalb ist bei Fig. 2 angenommen, daß sich die Strahlenquelle S (und die mit ihr über die Gantry verbundene, in Fig. 2 nicht dargestellte Detektoreinheit 16) auf der helixförmigen Bahn 17 bewegen, während der Untersuchungsbereich 13 (der - ebenso wie das darin befindliche Objekt - in Fig. 2 nicht dargestellt ist) ruht. Das von der Strahlenquelle S emittierte kegelförmige Strahlenbündel 4 trifft auf die jenseits des Untersuchungsbereichs befindliche Detektoreinheit 16, deren Mitte mit einem von der Strahlenquelle S ausgehenden, die Rotationsachse 14 senkrecht schneidenden Zentralstrahl zusammenfällt. Man kann sich das von der Sirahlenquelle 4 ausgehende Strahlenbündel als aus einer Vielzahl von Strahlenfächern zusammengesetzt denken, die sich in zur Rotationsachse 14 parallelen Ebenen befinden, die sich in der Strahlenquelle S schneiden. Obwohl dieses Strahlenbündel auch in anderen Ebenen fächerförmig gruppierte Strahlen enthält, sollen im folgenden nur solche Stiahlenkombinationen als Strahlenfächer bezeichnet werden, die - wie der Strahlenfächer 400 - in einer zur Rotationsachse 14 parallelen Ebene liegen. Die Meßdaten jedes einzelnen Strahlenfächers können von einer parallel zur Rotationsachse 14 verlaufenden Spalte von Detektorelementen der Detektoreinheit erfaßt werden.

Der Öffnungswinkel αₘₐₓ des Strahlenbündels 4 (in einer die Strahlquelle S enthaltenden, zur Rotationsachse 14 senkrechten Ebene) ist so bemessen, daß er mit seinen äußeren Fächern den Objektzylinder 13 gerade tangiert. Wenn αₘₐₓ = 30° ist, ist der Radius des Objektzylinders gerade halb so groß wie der Radius R der helixförmigen Bahn 17. Die Öffnung der Kollimatoranordnung 3 ist so gestaltet, daß zwei der Strahlenquelle gegenüberliegende, gegeneinander um die Strecke 3p versetzte (p entspricht dem Vorschub in z-Richtung während einer vollständigen Umdrehung der Strahlenquelle S) Windungen der Helix 17 mit den Strahlen am oberen und unteren Rand (bezogen auf die Darstellung in Fig. 2) des Strahlenbundels 4 zusammenfallen.

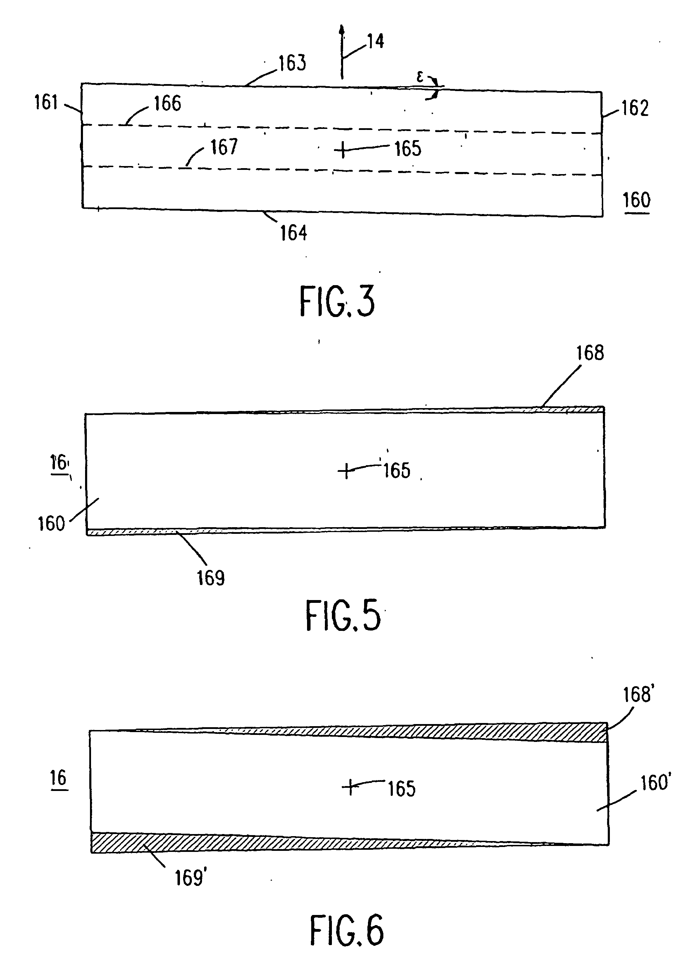

Der obere und untere Rand des erfindungsgemäßen Detektorfensters fällt mit der Projektion der Windungen der Helix (bzw. deren der Strahlenquelle gegenüberliegenden Abschnitten) auf die Detektoreinheit zusammen, d.h. die von der Strahlenquelle ausgehenden Verbindungsgeraden mit den erwähnten Rändern schneiden diese Windungen. Dies gilt auch, wenn das Detektorfenster sich nicht auf einem durch die Helix definierten Kreisbogen um die Rotationsachse befindet, sondern z.B. auf einem Kreisbogen um die Strahlenquelle S, was besondere Vorteile ergibt. In diesem Fall hat das Detektorfenster nicht mehr die in Fig. 3 gezeigte Form eines regulären Parallelogramms, sondern bildet ein verzerrtes Parallelogramm, dessen obere und untere Seite gekrümmt sind.

Fig. 3 stellt die Abwicklung des Detektorfensters 160 von dem durch die Helix 17 definierten Zylinder in die Zeichenebene dar. Die Abwicklung hat die Form eines Parallelogramms mit zur z-Richtung parallelen Seiten 161, 162 (deren Abstand voneinander um so größer ist, je größer der Öffnungswinkel αₘₐₓ des Strahlenbündels ist). Für die Länge dieser Seiten, d.h. die Höhe h des Detektorfensters, gilt h = 3p. Der obere und untere Rand 163 bzw. 164 des Detektorfensters schließt mit der Senkrechten auf die Rotationsachse 14 einen Winkel ε ein, der sich nach der Beziehung tan ε = p/2πR 2πR berechnen läßt, wobei R der Abstand der Strahlenquelle von der Rotationsachse ist. Dabei ist angenommen, daß die Vorschubgeschwindigkeit und die Rotationsgeschwindigkeit konstant sind. In Fig. 3 ist außerdem noch der Mittelpunkt 165 des Detektorfensters eingezeichnet, und mit den gestrichelten Linien 166 und 167 ist die Projektion der beiden Windungen bezeichnet, die zwischen den Windungen der Helix liegen, die mit dem oberen Rand 163 bzw. dem unteren Rand 164 zusammen fallen.

Jeder Punkt im Untersuchungsbereich wird bei seinem Eintritt in das kegelförmige Strahlenbündel 4 auf den unteren Rand 164 und bei seinem Austritt aus dem Strahlbündel auf den oberen Rand 163 projiziert. Es läßt sich zeigen, daß die Strahlenquelle um den betreffenden Punkt eine Drehung von genau 3π vollführt, während seine Projektion sich vom unteren Rand 164 des Detektorfensters sich zum oberen Rand 163 bewegt hat. Bezogen auf die Rotationsachse kann die von der Strahlenquelle ausgeführte Drehbewegung aber größer oder kleiner als 3π sein.

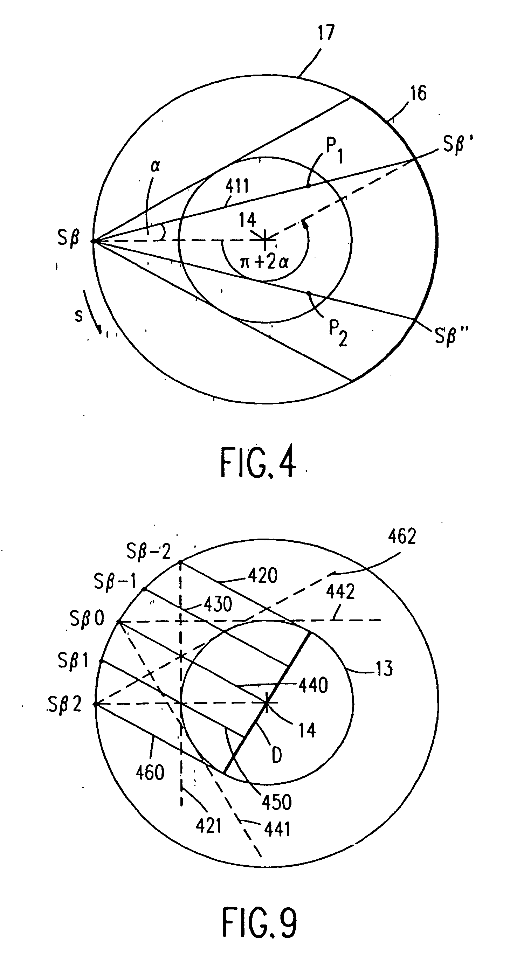

Dies ergibt sich aus Fig. 4, die die geometrischen Verhältnisse in einer Ansicht parallel zur z-Achse bzw. zur Rotationsachse 14 darstellt. Die Helix 17 geht dabei in einen Kreis über und die Rotationsachse 14 in einen Punkt, den Mittelpunkt dieses Kreises. Die Strahlenquelle bewegt sich auf der Bahn 17 in Richtung des Pfeiles s, und es ist ein Strahl 411 dargestellt, der einen Punkt P₁ in dem Moment durchstrahlt, in dem er in den Strahlenkegel eintritt. Nachdem sich die Strahlenquelle aus ihrer Position _{β} um eine volle Umdrehung und zusätzlich um den Winkel π+2α gedreht hat (insgesamt also um den Winkel 3π+α), befindet sie sich in einer Position S_{β}, in der die Projektion des oberen Punktes P₁ gerade den oberen Rand 163 des Detektorfensters passiert und die - bezüglich des Punktes P₁ - um genau 3π (gegenüber der anfänglichen Position der Srahlenquelle) gedreht ist.

Andererseits ist ein Punkt P₂ dargestellt, bei dessen Eintritt in das Strahlenbündel sich die Strahlenquelle ebenfalls in der Position S_{β} befindet, bei dessen Austritt sie jedoch die Position S_{β}. einnimmt. Auch hierbei hat sich die Strahlenquelle um den Punkt P₂ um genau 3π gedreht - um die Rotationsachse jedoch nur um den Winkel 3π-2α.

Der Punkt P₁ befindet sich daher proportional zum Drehwinkel (3π+2α) länger als der Punkt P₂ (mit dem Drehwinkel 3π-2α) in dem kegekförmigen Strahlenbündel. Das Verhältnis dieser Bestrahlungsdauern, das mit dem Signal/Rausch-Verhältnis für den betreffenden Punkt korreliert ist, ist im ungünstigsten Fall (für α=αₘₐₓ = 30°) 1,25:1 Würde sich die Strahlenquelle hingegen um die Punkte P₁ bzw. P₂ nur um den Winkel π drehen (wie bei dem eingangs erwähnten bekannten Computertomographen), dann würde dieses Verhältnis 2:1 betragen, was einer wesentlich stärkeren Ortsabhängigkeit des Signal/Rausch-Verhältnisses entsprechen würde.

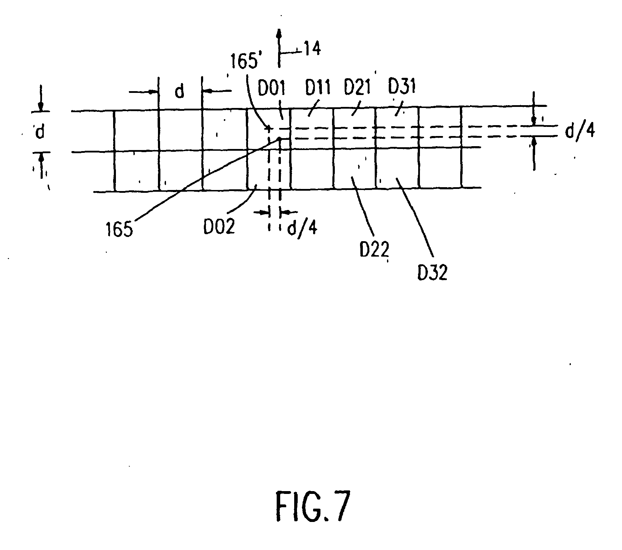

Ein gewisser Nachteil der in Fig. 3 dargestellten Abwicklung des Detektorfensters 160 ist seine Abweichung von der üblichen Rechteckform. Diese Abweichung ist noch ausgeprägter, wenn sich die Detektoreinheit auf einem Kreiszylinder befindet, dessen Zylinderachse durch die Strahlenquelle S verläuft und erst recht, wenn eine ebene Detektoreinheit verwendet würde. Fig. 5 zeigt daher eine Detektoreinheit, deren Abwicklung einem Rechteck entspricht und deren Abmessungen in z-Richtung so gewählt sind, daß sie zumindest das Detektorfenster 160 einschließen. Es verbleiben dann die in Fig. 5 schraffiert dargestellten Bereiche 168, 169 am oberen und unteren Detektorrand, die sich nach links bzw. nach rechts hin verjüngen.

Um trotz der von der Idealform des Detektorfensters 160 abweichenden Form der Detektoreinheit die gewünschte Akquisition der Meßdaten zu erreichen (nur die Meßdaten, bei denen jeder Meßpunkt die Strahlenquelle unter einem Winkel von genau 3π "sieht"), gibt es zwei Möglichkeiten, die auch in Kombination miteinander angewandt werden können:
a) Der Kollimator 3 (Fig. 3) wird so gestaltet, daß die Röntgenstrahlung nur das Detektorfenster 160 trifft, nicht aber die Randbereiche 168, 169.
b) Die Rekonstruktion erfolgt ausschließlich anhand der Meßwerte von Detektorelementen, die sich innerhalb des Detektorfensters 160 befinden. Die Meßwerte von sämtlichen Detektorelementen innerhalb der Streifen 168 und 169 werden ignoriert. Da sich die Lage des betektorfensters, 160 in bezug auf die Detektoreinheit 16 bei einer Untersuchung nicht ändert, ist der dafür erforderliche Softwareaufwand minimal.

Das in Fig. 5 dargestellte Rechteck ist größer als für die Aufnahme des Detektorfenster 160 erforderlich; d.h. ein Teil seiner Detektorelemente wird nicht ausgenutzt. Dieser unausgenutzte Anteil läßt sich verringern, wenn die Detektoreinheit von einer ersten Position (für eine kreisförmige Abtastbewegung), in der ihre Symmetrieachse parallel zur Rotatiohachse 14 verläuft, in eine zweite Position (für eine helixförmige Abtastbewegung) gekippt wird, in der ihre Symmetrieachse mit der Rotationsachse 14 einen spitzen Winkel bildet. Der obere und untere Rand 168, 169 sind dann in Bezug auf die Rotationsachse so geneigt wie die Windungen der Helix.

Fig. 6 zeigt die im Vergleich dazu die Abwicklung des Detektorfensters 160' (bzw der Detektoreinheit 16), das bei dem eingangs erwähnten bekannten CT-Gerät benutzt wird, bei der gleichen, rechteckigen Detektoreinheit 16 wie in Fig. 5. Man erkennt, daß die Steigung des oberen und unteren Randes des resultierenden Detektorfensters 160' eine um einen Faktor von ca. 3 größere Steigung haben als bei Fig. 5 bzw. Fig. 3. Außerdem ist ersichtlich, daß die für das Rechteck notwendigen Streifen 168' und 169', die die Detektorelemente enthalten, deren Meßwerte bei der Rekonstruktion nicht benötigt werden, breiter sind als die Streifen 168, 169 bei Fig. 5. Die Erfindung nutzt die Detektorfläche also besser aus.

Gleichwohl kann es sinnvoll sein, bei bestimmten CT-Untersuchungen wahlweise auch mit einem derartigen Detektorfenster zu arbeiten. Dazu wäre es erforderlich, die Rotationsgeschwindigkeit des Motors 2 (Fig. 1) für die Rotationsbewegung um den Faktor 3 herabzusetzen.

Bei dem vorstehenden Ausführungsbeispiel wurde davon ausgegangen, daß die in Rotationsrichtung gegeneinander versetzten Ränder des Detektorfensters durch die Windungen zweier Abschnitte der Helix definiert sind, die um die Strecke 3p in z-Richtung versetzt sind. Dieser Versatz kann aber auch 5p, 7p, 9p (allgemein (2n+1)p mit n≥ 1) betragen. Das Signal/Rausch-Verhältnis ist dabei noch weniger ortsabhängig, und es wird (bei einem Detektor mit einer rechteckigen Abwicklung der aktiven Detektorfläche) ein noch höherer Anteil ausgenutzt. Allerdings muß die Rotationsgeschwindigkeit (bzw. das Verhältnis zwischen Rotationsgeschwindigkeit und Vorschubgeschwindigkeit) dann um 5/3, 7/3 9/3 bzw. (2n+1)/3 höher sein.

Fig. 7 zeigt eine vorteilhafte Ausgestaltung der Detektoreinheit 16. Es sind zwei Zeilen von Detektorelementen D₀₁...D₃₁ sowie D₀₂...D₃₂ dargestellt. Das Detektorelement D₀₁ befindet sich in der mittleren Zeile und in der mittleren Spalte. Sein Mittelpunkt 165' fällt jedoch nicht mit dem Mittelpunkt 165 des Detektorfensters zusammen, der durch den Durchstoßpunkt einer von der Strahlenquelle S ausgehenden und die Rotationsachse 14 senkrecht schneidenden Geraden definiert ist. Zwischen diesen Punkten besteht vielmehr ein Versatz, der in Spaltenrichtung (bzw. in Richtung der Rotationsachse 14) und in Zeilenrichtung d/4 beträgt, wobei d der Breite bzw. Länge eines Detektorelementes entspricht.

Durch diesen Versatz in Zeilenrichtung um d/4 wird erreicht, daß nach einer halben Umdrehung der Abtasteinheit S, 16 nicht entlang derselben Strahlenpfade gemessen wird, sondern entlang von Strahlenpfaden, die um eine halbe Breite eines Detektorelements versetzt sind. Dadurch ergibt sich eine verbesserte Bildqualität. Durch den Versatz des Detektors in z-Richtung um d/4 verschieben sich die Spalten in z-Richtung nach einer halben Umdrehung ebenfalls um die halbe Breite eines Detektorelements, was ebenfalls zur Verbesserung der Bildqualität beiträgt.

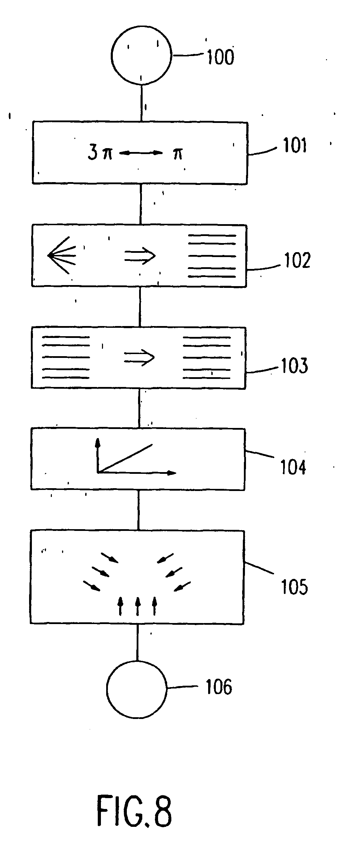

Im folgenden soll anhand des in Fig. 8 dargestellten Ablaufdiagramms die Verarbeitung der im Detektorfenster 160 akquirierten Meßdaten erläutert werden. Dabei wird davon ausgegangen, daß die Meßfläche der Detektoreinheit rechteckig ist entsprechend Fig. 5 bzw. Fig. 6 und daß der Benutzer wählen kann, ob er mit der erfindungsgemäßen Datenakquisition arbeiten will, bei der jeder Punkt im Untersuchungsbereich die Strahlenquelle bei seiner Passage durch den Strahlenkegel aus einem Winkelbereich von 3π (5π, 7π....) sieht, oder ob er von der eingangs genannten bekannten Datenakquisition ausgeht.

Nach der Initialisierung (Block 100) werden im Block 101 Meßdaten akquiriert, wobei der Benutzer vorgeben kann, ob während eines Vorschubs in z-Richtung entsprechend der Höhe h des Detektorfensters die Strahlenquelle um den Winkel 3π (5π, 7π ...) oder um den Winkel π rotieren soll. Diese Wahl hat - wie vorstehend erläutert - auf die Datenakquisition und die Qualität der Rekonstruktion zwar einen deutlichen Einfluß, jedoch muß das eigentliche Rekonstruktionsverfahren nur geringfügig modifiziert werden. Es wird daher Bezug genommen auf die Beschreibung des Rekonstruktionsverfahrens in der WO 98/30980.

Demgemäß erfolgt zunächst ein Rebinning, bei dem in einem ersten Schritt - 102 - Gruppen von in parallelen Ebenen liegenden Strahlenfächern gebildet werden. Wie anhand von Fig. 2 erläutert, kann man sich das kegelförmige Strahlenbündel als aus Strahlenfächern - wie den Fächer 400 in Fig. 2 - zusammengesetzt denken, die in zur Rotationsache 14 parallelen Ebenen liegen. In der Draufsicht gemäß Fig. 4 gehen diese Ebenen bzw. Strahlenfächer in Geraden über. Es werden nun die Strahlenfächer zu jeweils einer Gruppe zusammengefaßt, die in zueinander (und zur Rotationsachse 14) parallelen Ebenen liegen und die aus verschiedenen Strahlenquellenpositionen aufgenommen sind (die bei einer einzigen Strahlenquellenposition sich ergebenden Strahlenfächer sind ja nicht zueinander parallel).

Fig. 9 zeigt eine einzige Gruppe solcher Strahlenfächer, wobei die Zeichenebene die Rotationsachse 14 - ebenso wie bei Fig. 4 - senkrecht schneidet. In Fig. 9 sind aufeinander folgende Positionen der Strahlenquelle mit S_{β-2}...S_{β0}...S_{β2} bezeichnet. Von jeder dieser Strahlenquellenpositionen geht ein Strahlenfächer 420...440...460 aus, der den Untersuchungsbereich 13 durchsetzt und der in einer Ebene liegt, die parallel zu der Ebene ist, die durch die mittlere Detektorposition S_{β0} und die Rotationsachse 14 definiert wird. Für die zur gleichen Gruppe gehörenden Strahlenfächer gilt, daß die Summe des Fächerwinkels a (das ist der Winkel, den die Ebene des Strahlenfächers mit der durch die zugehörige Strahlenquellenposition und die Rotationsachse 14 definierten Ebene einschließt) und des Winkels, den die Strahlenquelle mit der Rotationsachse 14 einschließt, konstant ist. Wenn in einer Strahlenquellenposition kein Strahlenfächer diese Bedingung exakt erfüllt, wird aus beiderseits der Ebene mit der gesuchten Orientierung liegenden und in der betreffenden Strahlenquellenposition erzeugten Strahlenfächern ein in dieser Ebene befindlicher Strahlenfächer durch Interpolation ermittelt.

Außerdem sind noch einige Strahlenfächer, z.B. 421, 441 bzw. 442, 462 dargestellt, die jeweils am Rande eines Strahlenkegels liegen (und somit den Untersuchungsbereich tangieren). Diese Strahlenfächer gehören jedoch nicht zu der in Fig. 9 dargestellten Gruppe und sind daher gestrichelt dargestellt - und nicht in ausgezogenen Linien wie die zur Gruppe gehörenden Strahlenfächer.

Fig. 9 zeigt darüber hinaus ein zu der dargestellten Gruppe gehöriges virtuelles Detektorfenster D, das senkrecht zu den Ebenen der Strahlenfächer und durch die Rotationsachse 14 verläuft. Das virtuelle Detektorfenster D ist rechteckig und hat die Höhe h/2 bzw. 1,5p, wobei die Mitte des virtuellen Detektorfensters durch das Lot von der mittleren Strahlenquellenposition S_{β0} auf die Rotationsachse definiert ist. Obwohl die Strahlenquellenposition S_{β-2} und S_{β2} in z-Richtung gegenüber der mittleren Strahlenquellenposition S_{β0} in z-Richtung versetzt sind, läßt sich zeigen, daß die oberen und unteren Rasdstrahlen der Strahlenfächer 420 und 460 exakt mit dem oberen und unteren Rand des virtuellen Detektorfensters D zusammenfallen. Dies beruht darauf, daß die Strahlenfächer 460 bzw. 420, die beiderseits der mittleren Strahlenquellenposition S_{β} liegen, von rechts bzw. links der Mitte liegenden Spalten des realen Detektorfensters 160 erfaßt werden, die in z-Richtung höher bzw. niedriger liegen als die Spalte, die den von S_{β0} ausgehenden Strahlenfächer erfaßt. Jeder Strahlenfächer bedeckt daher eine Spalte des virtuellen Detektorfensters D.

Im Schritt 102 werden die (in zur Rotationsachse parallelen Ebenen befindlichen) Strahlenfächer je einer der verschiedenen Gruppen zugeordnet. Wenn man von den Strahlenquellenpositionen am Anfang und am Ende der helixförmigen Abtastbahn 17 absieht, gibt es für jede Strahlenquellenposition eine Gruppe. Zu jeder Gruppe gehört ein virtueller Detektor D, dessen ebene rechteckige Fläche senkrecht zu den Ebenen verläuft, in denen sich die zu dieser Gruppe gehörenden Strahlenfächer befinden.

Nachdem auf diese Weise für wenigstens eine Gruppe alle zugehörigen Strahlenfächer erfaßt worden sind, erfolgt im Schritt 103 der zweite Teil des Rebinning, nämlich die Uminterpolation bzw. das Resampling. Wie Fig. 9 zeigt, nimmt der Abstand der parallelen Ebenen, in denen sich die zu einer Gruppe befindlichen Strahenfächer befinden, von der Mitte nach außen hin ab. Deshalb werden im Schritt 103 für ein regelmäßiges kartesisches Gitter auf dem virtuellen Detektorfenster D die zugehörigen Meßdaten aus den im Schritt 102 den einzelnen Gruppen zugeteilten Meßdaten ermittelt, vorzugsweise durch Interpolation. Damit ist ein Rebinning einer parallelen Fächergeometrie auf eine rechteckige Fläche mit regelmäßig verteilten Gitterpunkten erfolgt, was die nachfolgende Verarbeitung ganz wesentlich erleichtert.

Anschließend erfolgt im Schritt 104 eine eindimensionale Filterung. Bei dem durch die Schritte 102 und 103 erfolgenden Rebinning ist dafür lediglich ein einfaches eindimensionales ortsunabhängiges Filter erforderlich, das die Meßdaten in dem virtuellen Detektorfenster D in Zeilenrichtung, d.h. senkrecht zur Rotationsachse 14, filtert. Dieses Filter hat eine linear mit der Frequenz abnehmende Dämpfung. Die Filterung kann im Prinzip dadurch erfolgen, daß die aus dem Rebinning resultierenden Meßdaten einer Faltung (Konvolution) mit einem geeigneten eindimensionalen Filterkernel unterzogen werden.

Ein geringerer Aufwand ergibt sich allerdings, wenn die Filterung im Schritt 104 zunächst eine Fouriertransformation vorsieht, wonach die auf diese Weise in den Ortsfrequenzraum transponierten Daten einer rampenförmigen Filterung in Zeilenrichtung unterzogen werden, um danach durch eine inverse Fouriertransformation wieder in den Ortsraum rücktransformiert zu werden.

Im Schritt 105 werden die gefilterten Daten einer jeden Gruppe bzw aus jedem der virtuellen Detektorfenster wieder in den Untersuchungsbereich rückprojiziert, d.h. die gefilterten Meßdaten werden gruppenweise und entlang der gleichen (durch den Schritt 103 gegebenenfalls geringfügig modifizierten) Strahlenpfade in den Untersuchungsbereich rückprojiziert, längs derer sie akquiriert wurden. Die Absoptionswerte für die einzeln Voxel des Untersuchungsbereichs ergeben sich aus der Überlagerung sämtlicher (gefilterter) Meßdaten, bei deren Akquisition das betreffende Voxel auf das Detektorfenster 160 projiziert wurde. Für jedes rekonstruierte Voxel ergeben sich dabei Beiträge von Strahlenfächern bzw. Gruppen aus einem Winkelbereich von 3π - bzw. von (2n+1) π, anstatt - wie bei dem bekannten Verfahren - aus einem Winkelbereich von π.

Auf diese Weise kann schon während der Akquisition der Meßdaten mit der Rekonstruktion der Absorptionsverteilung im Untersuchungsbereich begonnen werden. Akquisition und Rekonstruktion können dann zeitlich nebeneinander durchgeführt werden, bis die Akquisition - zu einem frei wählbaren Zeitpunkt - abgebrochen und die Rekonstruktion mit den bis dahin gewonnenen Meßdaten komplettiert wird. Das Verfahren endet dann (Block 106).

Die Absorptionsverteilung im Untersuchungsbereich kann aus den mit dem erfindungsgemäßen Detektor akquirierten Meßdaten auch auf andere Weise rekonstruiert werden. Ein geeignetes anderes Rekonstruktionsverfahren ist aus Phys. Med. Biol. 43 (1998) 1015-1024 bekannt.

Dabei wird nach der Methode von Grangeat für jede Ebene, die das Objekt schneidet, die erste Ableitung des Flächenintegrals aus Segmenten berechnet, und die partiellen Ergebnisse werden in einer Summe akkumuliert. Diese Daten bilden die erste radiale Ableitung des Radonraums, die mit bekannten Verfahren in den Objektraum invertiert werden kann. Dabei sind in Abhängigkeit von der Zahl der Schnittpunkte der zu rekonstruierenden Ebene mit der Helix, auf der der Untersuchungsbereich abgetastet wird, folgende Unterfälle bei der Verarbeitung der mit dem erfindungsgemäßen Detektor akquirierten Meßdaten zu unterscheiden:
a) Kein Schnittpunkt,
   In diesem Fall gibt es keine Information, weil die zu rekonstruierende Ebene das Objekt nicht schneidet.
b) Ein Schnittpunkt
   In diesem Fall werden bei dem bekannten Verfahren ebenso wie bei der Erfindung die gleichen Meßdaten akquiriert.
c) Es gibt eine ungerade Anzahl von Schnittpunkten
   In diesem Fall wird die Rekonstruktion wie bei dem bekannten Verfahren durchgeführt und das Ergebnis wird durch 3 dividiert (wenn die Dptektorabmessung h dem Dreifachen des Abstandes der Detektorwindungen entspricht).

## Patentansprüche

1. Computertomograph mit
- einer Abtasteinheit, die eine Strahlenquelle (S) und eine damit verbundene Detektoreinheit (16) zur Erfassung eines von der Strahlenquelle emittierten, kegelförmigen Strahlenbündels nach dem Durchgang durch einen Untersuchungsbereich (13) bzw. durch ein darin befindliches Objekt umfaßt,
- einer Antriebsanordnung (2,5) zur Erzeugung einer eine Rotation um eine Rotationsachse (14) und einen Vorschub in Richtung parallel zur Rotationsachse umfassenden Relativbewegung in Form einer Helix (17) zwischen der Abtasteinheit (S,16) und dem Untersuchungsbereich (13) bzw. dem Objekt
- und mit einer Rekonstruktionseinheit (10) zur Rekonstruktion der räumlichen Verteilung der Absorption innerhalb des Untersuchungsbereiches (13) aus den von der Detektoreinheit (16) innerhalb eines durch die Helix (17) definierten Detektorfensters (160) akquirierten Meßdaten
**dadurch gekennzeichnet, daß** die Verbindungsgeraden der Strahlenquelle (S) mit den beiden in Richtung der Rotationsachse (14) gegeneinander versetzten Rändern (163, 164) des Detektorfensters (160) zwei in Richtung der Rotationsachse um die Strecke (2n+1)p versetzte Abschnitte der Helix (17) schneiden, wobei n eine ganze Zahl ≥ 1 ist und p dem achsialen Versatz zweier benachbarter Windungen der Helix entspricht.

2. Computertomograph nach Anspruch 1,
**dadurch gekennzeichnet, daß** n=1 ist.

3. Computertomograph nach Anspruch 1, wobei die Detektoreinheit (16) in Zeilen und Spalten angeordnete Detektorelemente (D₀₁ , D₀₂ ...) umfaßt,
**dadurch gekennzeichnet, daß** die Mitte der Zeilen gegenüber einer die Strahlenquelle und die Rotationsachse enthaltenden Ebene um ein Viertel der Breite (d) eines Detektorelements versetzt ist.

4. Computertomograph nach Anspruch 1, wobei die Detektoreinheit in Zeilen und Spalten angeordnete Detektorelemente umfaßt,
**dadurch gekennzeichnet, daß** die Mitte der Spalten gegenüber einer die Strahlenquelle enthaltenden und zur Rotationsachse senkrechten Ebene die Hälfte der Breite eines Detektorelements versetzt ist.

5. Computenomograph nach Anspruch 1,
**dadurch gekennzeichnet, daß** die Antriebsanordnung (2, 5) zwei Betriebsmodi aufweist wobei in dem einen Betriebsmodus das Verhältnis zwischen der Rotationsgeschwindigkeit und der Vorschub-Geschwindigkeit um einen Faktor 2n+1 kleiner ist als im anderen Betriebsmodus, sodaß im einen,Betriebsmodus jeder Punkt (P₁)im Untersuchungsbereich (13) von der Strahlenquelle (S) aus einem Winkelbereich von π und im anderen Betriebsmodus aus einem Winkelbereich von (2n+1)π von der Strahlenquelle bestrahlt wird.

6. Computertomograph nach Anspruch 1,
**gekennzeichnet durch**,
a) Mittel zum Rebinning der Meßdaten zu einer Anzahl von Gruppen, wobei jede Gruppe mehrere zur Rotationsachse parallele Ebenen umfaßt, in denen sich je ein Strahlenfächer befindet,
b) Mittel zur Filterung der **durch** das Rebinning erzeugten Daten einer jeden Gruppe in Richtung senkrecht zur Rotationsachse,
c) Rekonstruktion der räumlichen Verteilung der Absorption **durch** die Rückprojektion der gefilterten Daten von mehreren Gruppen.

## Claims

1. A computer tomograph which includes
- a scanning unit which includes a radiation source (S) and a detector unit (16) which is connected thereto in order to detect a conical radiation beam, emitted by the radiation source, after its passage through an examination zone (13) or an object present therein,
- a driving device (2, 5) for producing a relative motion in the form of a helix (17), consisting of a rotation about an axis of rotation (14) and an advance in the direction parallel to the axis of rotation, between the scanning unit (S, 16) and the examination zone (13) or the object,
- and a reconstruction unit (10) for reconstructing the spatial distribution of the absorption within the examination zone (13) from the measuring data acquired by
the detector unit (16) within a detector window (160) defined by the helix (17), **characterized in that** the connecting lines from the radiation source (S) to the two edges (163, 164) of the detector window (160), which are mutually offset in the direction of the axis of rotation (14), intersect two segments of the helix (17) which are offset by the distance (2n+1)p in the direction of the axis of rotation, where n is an integer number ≥ 1 and p corresponds to the axial offset between two neighboring turns of the helix.

2. A computer tomograph as claimed in Claim 1,
**characterized in that** n = 1.

3. A computer tomograph as claimed in Claim 1, in which the detector unit (16) includes detector elements (D₀₁, D₀₂...) which are arranged in rows and columns,
**characterized in that** the center of the rows is offset by one quarter of the width (d) of a detector element relative to a plane containing the radiation source and the axis of rotation.

4. A computer tomograph as claimed in Claim 1, in which the detector unit includes detector elements which are arranged in rows and columns,
**characterized in that** the center of the columns is offset by half the width of a detector element relative to a plane containing the radiation source and extending perpendicularly to the axis of rotation.

5. A computer tomograph as claimed in Claim 1,
**characterized in that** the driving device (2, 5) has two modes of operation, the ratio of the rotary speed to the transport speed being a factor 2n+1 smaller in one operating mode in comparison with the other operating mode, so that in one operating mode each point (P₁) in the examination zone (13) is irradiated by the radiation source (S) from an angular range π, whereas it is irradiated thereby from an angular range (2n+1)π in the other mode of operation.

6. A computer tomograph as claimed in Claim 1,
**characterized in that** it includes:
a) means for rebinning the measuring data so as to form a number of groups, each group including a plurality of planes which extend parallel to the axis of rotation and in which a respective fan beam is present,
b) means for filtering the data of each group, formed by the rebinning, in the direction perpendicular to the axis of rotation,
c) reconstruction of the spatial distribution of the absorption by backprojection of the filtered data of a plurality of groups.

## Revendications

1. Tomographe informatisé avec :
- une unité de balayage qui comprend une source de rayonnement (S) et une unité détectrice (16) associée pour la saisie d'un faisceau de rayonnement conique émis par la source de rayonnement après le passage par une zone à examiner (13) ou par un objet qui s'y trouve,
- un dispositif d'entraînement (2, 5) pour la production d'un mouvement relatif comprenant une rotation autour d'un axe de rotation (14) et une avance dans une direction parallèle à l'axe de rotation entre l'unité de balayage (S, 16) et la zone à examiner (13) ou l'objet sous la forme d'une hélice (17)
- et avec une unité de reconstruction (10) pour la reconstruction de la distribution spatiale de l'absorption dans la zone à examiner (13) à partir des données de mesure acquises par l'unité de détecteur (16) dans une fenêtre du détecteur (160) définie par l'hélice (17),
**caractérisé en ce que** les droites de liaison de la source de rayonnement (S) avec les deux bords (163, 164) de la fenêtre du détecteur (160) décalés l'un par rapport à l'autre dans la direction de l'axe de rotation (14) coupent deux segments de l'hélice (17) décalés dans la direction de l'axe de rotation de la zone (2n+1)p, n étant un nombre entier ≥ 1 et p le déport axial de deux enroulements voisins de l'hélice.

2. Tomographe informatisé selon la revendication 1,
**caractérisé en ce que** n = 1.

3. Tomographe informatisé selon la revendication 1, dans lequel l'unité de détecteur (16) comprend des éléments détecteurs (D₀₁, D₀₂, ...) disposés en lignes et colonnes,
**caractérisé en ce que** le milieu des lignes est décalé par rapport à un plan contenant la source de rayonnement et l'axe de rotation d'un quart de la largeur (d) d'un élément détecteur.

4. Tomographe informatisé selon la revendication 1, dans lequel l'unité de détecteur comprend des éléments détecteurs disposés en lignes et colonnes,
**caractérisé en ce que** le milieu des lignes est décalé par rapport à un plan contenant la source de rayonnement et l'axe de rotation de la moitié de la largeur d'un élément détecteur.

5. Tomographe informatisé selon la revendication 1,
**caractérisé en ce que** le dispositif d'entraînement (2, 5) présente deux modes de fonctionnement, dans lequel le rapport entre la vitesse de rotation et la vitesse d'avance est inférieur d'un facteur 2n+1 1 dans un mode de fonctionnement par rapport à l'autre mode de fonctionnement de telle sorte que chaque point (P₁) dans la zone à examiner (13) soit exposé par la source de rayonnement (S) d'une zone angulaire π dans un mode de fonctionnement et par la source de rayonnement d'une zone angulaire de (2+1)π dans l'autre mode de fonctionnement.

6. Tomographe informatisé selon la revendication 1,
**caractérisé par**
a) des moyens pour le rebinning des données de mesure à un nombre de groupes, chaque groupe comprenant plusieurs plans parallèles à l'axe de rotation dans lesquels se trouve respectivement un éventail de rayons ;
b) des moyens de filtrage des données produites par le rebinning de chaque groupe dans une direction perpendiculaire à l'axe de rotation ;
c) la reconstruction de la distribution spatiale de l'absorption par la rétroprojection des données filtrées par plusieurs groupes.
